(19) Europäisches Patentamt
European Patent Office
Office européen des brevets

(11) **EP 2 887 351 A1**

(12) **EUROPEAN PATENT APPLICATION**

(43) Date of publication:
**24.06.2015 Bulletin 2015/26**

(51) Int Cl.:
*G10L 25/75* [(2013.01)]    *G10L 13/02* [(2013.01)]

(21) Application number: **13005905.8**

(22) Date of filing: **18.12.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Karlsruher Institut für Technologie**
**76131 Karlsruhe (DE)**

(72) Inventors:
• **Schultz, Tanja**
**69121 Heidelberg (DE)**
• **Wand, Michael**
**76227 Karlsruhe (DE)**
• **Janke, Matthias**
**76137 Karlsruhe (DE)**

(74) Representative: **Müller-Boré & Partner**
**Patentanwälte PartG mbB**
**Friedenheimer Brücke 21**
**80639 München (DE)**

(54) **Computer-implemented method, computer system and computer program product for automatic transformation of myoelectric signals into audible speech**

(57) In one aspect, the present application is directed to a computer-implemented method, a computer program product, and a computer system for automatic transformation of myoelectric signals into speech output corresponding to audible speech. The computer-implemented method may comprise:

capturing, from a human speaker, at least one myoelectric signal representing speech;
converting at least part of the myoelectric signal to one or more speech features; and
vocoding the speech features to generate and output the speech output corresponding to the myoelectric signal.

Figure 1

# EP 2 887 351 A1

## Description

<u>Technical Field</u>

The description is directed generally to machine-based generation of speech and, in particular, to a computer-implemented method, a computer system, and a computer program product for automatic transformation of myoelectric signals into audible speech.

<u>Background</u>

Improving man-machine interactions and the desire to support any kind of human to human communication, human to machine communication, and/or cooperation among humans using (assistive) computing technology has led to the development of machines capable of processing, recognizing, and/or understanding (natural language) spoken input. Natural language spoken input is herein after referred to as speech or utterance of spoken text.

Speech recognition systems have been built which provide good results when speech is spoken audibly (i.e. a sound pressure wave is produced which transports audible sounds and thus not silent) with normal vocal effort in relatively quiet environments.

However, despite the various benefits a conventional speech-driven interface provides to humans, there are several drawbacks. Audible speech signals prohibit a confidential conversation with or through a device. Also, talking can be extremely disturbing to others, for example in libraries, during meetings, etc. Speech recognition performance can degrade drastically in adverse environmental conditions such as in restaurants, cars, or trains. Performance is also poor when sound production limitations occur, such as under water, under a firefighter, and/or pilot mask. Further, conventional speech-driven interfaces cannot be used by speech disabled people, for example those without vocal cords.

To overcome drawbacks of such speech recognition systems, it has been recognized that myoelectric signals captured from articulatory muscles in the body, face, neck, mouth, etc. of a speaker substantially include sufficient information to discriminate sounds and words. This holds even when speech is produced (for example when the words are spoken) non-audibly (i.e. silent, when no audible signal is produced).

Said observations have been used to develop myoelectric signal-based automatic speech recognition systems for myoelectric signal-based processing of speech and/or the recognition of sounds and words into a textual representation. Such a myoelectric signal-based speech recognition system basically operates by capturing myoelectric signals from a user (also referred to herein as a speaker), whereby contractions of muscles are converted into an electric current. The electric current is then converted into a digital signal. A digital device is used to transform the digital signal into a written representation using an automatic speech recognition method. An audible output can then be generated from the written representation using a speech synthesis method.

However, such kinds of myoelectric signal-based speech recognition systems have several drawbacks. For example, since speech recognition basically relies on pronunciation dictionaries to guide and restrict the search process, words which are new or unknown to the system cannot be recognized and thus not synthesized. Similarly, idioms cannot be handled. Also, speech recognition output is not free of errors, even in ideal situations a misrecognized word may occur. Further, it is very time and cost consuming to support speech recognition for potentially every spoken language and hence myoelectric signal-based speech recognition.

Hence, there is a need to provide more robust and flexible systems and methods for automatic generation of audible speech from myoelectric signals.

<u>Summary</u>

According to one general aspect, a computer-implemented method for automatic transformation of myoelectric signals into speech output corresponding to audible speech. The method may comprise:

capturing, from a human speaker, at least one myoelectric signal representing speech;
converting at least part of the myoelectric signal to (a representation of) one or more speech features; and
vocoding the speech features to generate and output the speech corresponding to the myoelectric signal.

The speech output may comprise an audio file in any format (e.g. mpeg, wav, pcm), an electrical signal, a stream of speech features, an output of an audible signal through a loud speaker, etc.

Speech features may comprise any computer-based representation from which speech output, as defined above, may be generated, including a representation of the speech waveform as a function of time, stored in digital or analog form, and/or a representation of speech based on temporal features, such as a windowed speech signal and/or features such as signal energy, mean, zero-crossing rate, and/or a representation of speech based on its frequency or spectral contents (spectral features), for example, as a spectrogram, as spectral coefficients and/or another representation derived from the speech frequencies, and/or a representation of vocal tract properties, such as linear prediction coefficients, and/or any combination thereof. Speech features further include any representation of prosodic features, such as the fundamental frequency, i.e. the frequency of the vocal chords, the signal energy, and/or the intonation contours of an utterance.

A myoelectric signal may by be captured from articulatory activity of a speaker (or user) representing speech. The myoelectric signal may result from audible and/or silent speech produced by the speaker.

The myoelectric signal may be, during a preprocessing step, digitalized for the conversion to (a) corresponding (speech representation of) speech features. In order to convert the myoelectric signal into a representation of speech features, the raw analog or digital myoelectric signal may be processed as follows:

In an aspect, the representation may be a spectral representation of speech comprising spectral features, temporal features, and/or spectro-temporal features.

In another aspect, the method may further comprise computing an estimate for a fundamental frequency of the spectral representation of speech.

The fundamental frequency is defined as the frequency with which the vocal chords of a person vibrate when producing audible speech. Furthermore, fundamental frequency information can also comprise information about whether the fundamental frequency exists at any point of time. In this case the speaker is producing a voiced sound. Likewise, whether the fundamental frequency does not exist at any point of time (i.e. the vocal chords do not vibrate). In this case, the speaker may produce an audible unvoiced sound, whispered and/or silent speech, or is completely mute.

In the conversion step, the fundamental frequency information determines the intonation of the speech which is output by the system. When converting silent or whispered into audible speech, the fundamental frequency is estimated from the input myoelectric signal, which may be done by using the GMM-based mapping technique and/or by any other method, including by incorporating knowledge about the speech intonation process.

In yet another aspect, joint feature vectors resulting from a prior training phase on myoelectric signals and corresponding audible signals may be used to predict the representation of speech for the myoelectric signal.

In yet another aspect, at least one myoelectric signal may be captured from the speaker's head, throat, face, mouth, chest, and/or neck using an array-based electrode system.

In yet another aspect, the speech may be produced by the speaker comprising normally articulated speech, whispered speech, murmured speech, speech that is barely or not audible to a bystander, and/or silently mouthed speech.

In yet another aspect, the method may further comprise: receiving feedback from the speaker and/or a receiver on the speech output through multiple modalities comprising audible signals, visible signals, and/or tactile signals.

In another general aspect there is provided a computer-program product comprising computer readable instructions, which when loaded and run in a computer system and/or computer network system, cause the computer system and/or the computer network system to perform a method as described.

In yet another general aspect, a computer system for automatic transformation of myoelectric signals into audible speech is provided. The system may comprise:

a capturing device operable to capture, from a human speaker, at least one myoelectric signal representing speech;
a silent/audio conversion component operable to convert at least part of the myoelectric signal to a representation of one or more speech features; and
a vocoding component operable to vocode the speech features to generate and output the speech output corresponding to the myoelectric signal.

In another aspect, the computer system is operable to implement a processor to perform a method as described.

The subject matter described in this specification can be implemented as a method or as a system or using computer program products, tangibly embodied in information carriers, such as a CD-ROM, a DVD-ROM, a semiconductor memory, signal and/or data stream, and a hard disk. Such computer program products may cause a data processing apparatus to conduct one or more operations described in this specification.

In addition, the subject matter described in this specification can also be implemented as a system including a processor and a memory coupled to the processor. The memory may encode one or more programs that cause the processor to perform one or more of the method steps or actions described in this specification. Further the subject matter described in this specification can be implemented using various MRI machines.

Details of one or more implementations are set forth in the accompanying exemplary drawings and exemplary description below. Other features will be apparent from the description and drawings, and from the claims.

Brief Description of the Drawings

Figure 1 shows an exemplary system and method for transforming myoelectric signals into corresponding audible speech.

Figure 2 shows an exemplary system and method for acquisition and/or processing of myoelectric signals.

Figure 3 shows an exemplary hardware device usable for the acquisition of myoelectric signals.

Figures 4A, 4B, and 4C show exemplary positioning of electrodes and/or electrode arrays and exemplary electrode arrays usable for the acquisition of myoelectric signals.

Figure 5 shows and exemplary method and system for processing myoelectric signals.

Figure 6 shows and exemplary silent mapping method.

Figure 7 shows an exemplary GMM (Gaussian mixture model) conversion method.

Figure 8 shows an exemplary method for generating prosodic speech features and a prosody subsystem using GMM and SVM (support vector machine) discrimination.

Figure 9 shows an exemplary computer system for implementing computer systems and computer-implemented methods as shown in Figs. 1 to 8.

Detailed Description

In the following, a detailed description of examples will be given with reference to the drawings. It should be understood that various modifications to the examples may be made. In particular, elements of one example may be combined and used in other examples to form new examples.

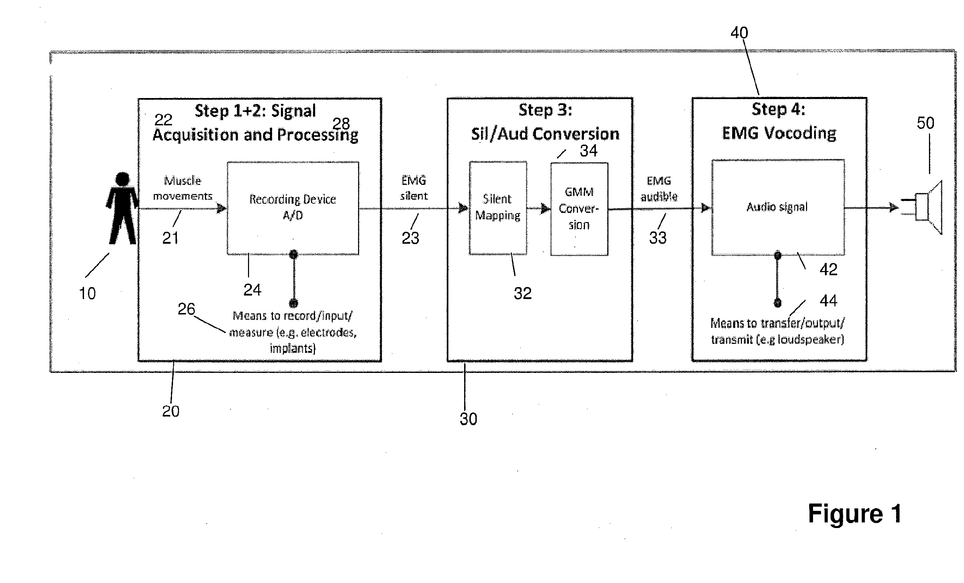

**Figure 1** shows an exemplary computer implementation of a method and system transforming at least one myoelectric signal 21 resulting from articulatory activity of a speaker 10 into corresponding audible speech 50. A myoelectric signal 21 basically relates to an electrical impulse that produces contraction of muscle fibers in the body of the speaker 10 and therefore represents an articulatory activity of the speaker 10. Preferably, a myoelectric signal 21 represents speech of the speaker 10 and is captured from the speaker's 10 body, face, throat, mouth, and/or neck. Myoelectric signals 21 captured from the speaker's 10 body, face, throat, mouth, and/or neck may have frequencies ranging from few Hertz (Hz) to about 500 Hz and voltages ranging from approximately 10 microvolt to 1 millivolt. Myoelectric signals may be detected by placing (non-invasively) surface electrodes and/or surface electrode arrays on the skin, preferably, of the body, face, throat, mouth, and/or neck of a speaker 10. Further the electrodes and/or arrays of electrodes may be imprinted onto the skin, injected under the skin of the speaker 10 and/or permanently implanted.

The shown method comprises steps of signal acquisition and processing 20, silent/audio conversion 30, and/or vocoding of myoelectric signals 40. The steps may be implemented and performed by corresponding software and/or hardware components of a computer or computer system referred to herein after as a signal acquisition and processing component 20, a silent/audio conversion component 30, and/or a vocoding component 40.

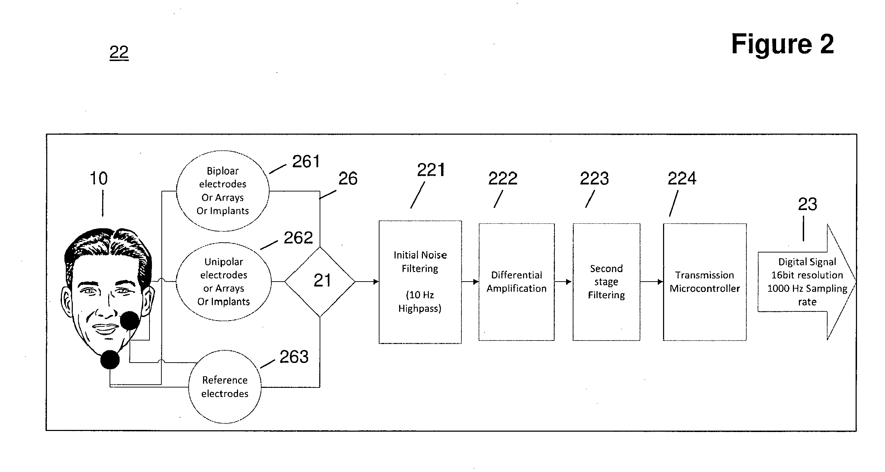

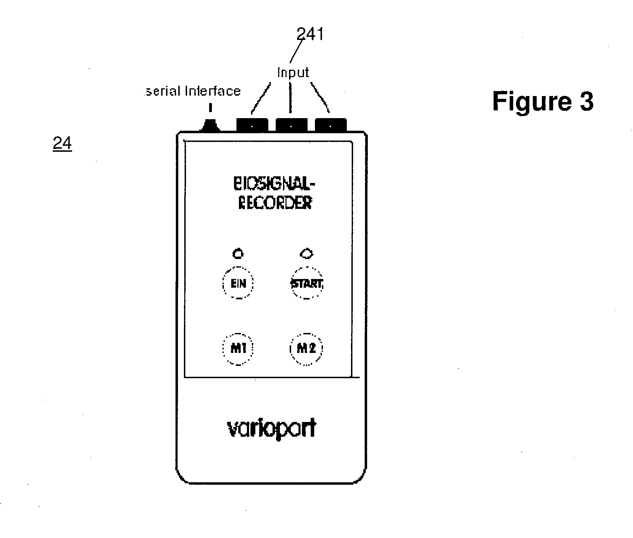

Signal acquisition and processing 20 is further described below with reference to figures 2 to 5. The Signal acquisition and processing 20 may be divided into a first step of (myoelectric) signal acquisition 22 and a second step of (myoelectric) signal processing 28 being implemented by corresponding computer components. During signal acquisition 22 a myoelectric signal 21 representing speech produced by a speaker 10, is retrieved by capturing the myoelectric signal 21 from the speaker's 10 body, face, throat, mouth, and/or neck using a capturing device 26 such as one or more electrodes and/or at least one array of electrodes. A recording device 24 may be further used to implement one or more steps of the signal acquisition and processing 20. The recording device 24 is described in greater detail below with reference to figure 3.

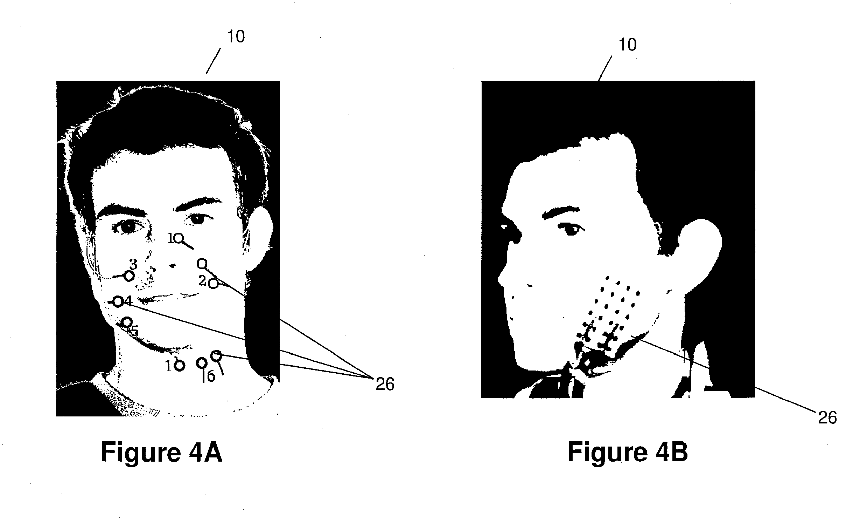

The capturing device 26 for capturing myoelectric signals 21 from a speaker 10 may comprise a single surface electrode, arrays of surface electrodes, and/or other types of electrode setups at the surface of the skin, imprinted onto the skin, and/or injected into the skin, or implanted underneath the surface of the speaker's 10 skin. The capturing device 26 converts an ionic current generated by muscle activity of the speaker 10 into an electric current. Example implementations of the capturing device 26 including electrodes and/or arrays of electrodes and positioning of the capturing device 26 at a speaker 10 are described below with reference to figures 4A to 4C.

During signal processing 28, the resulting electric current of a captured myoelectric signal 21 is recorded, amplified, filtered, and/or converted into a corresponding digital myoelectric signal 23. Said computation may comprise transmitting the myoelectric signal 21 to a corresponding digital myoelectric signal 21 and/or removing artifacts resulting from technical, environmental, or human factors, causing adverse conditions due to technical issues, due to unrelated muscle movements, due to the environment and/or due to the speaker's 10 behavior and properties.

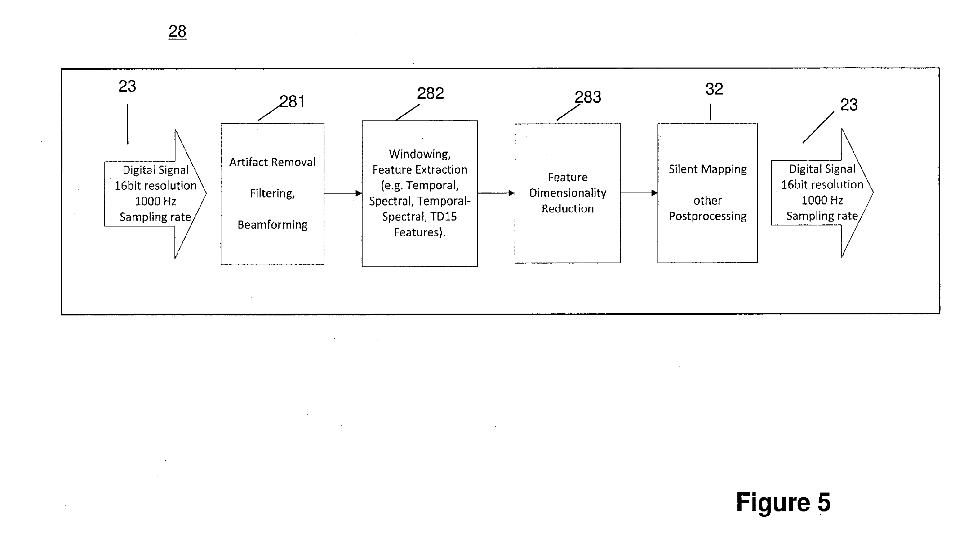

Signal acquisition 22 is described in further detail with reference to figure 2 and signal processing 28 is described in further detail with reference to figure 5.

During silent/audible conversion 30 at least one captured and digitalized myoelectric signal 23 is converted to one or more speech feature representations (of a plurality of possibly different speech features) by applying a silent mapping method 32 and/or a GMM conversion method 34. Speech features are defined as any computer-based representation from which speech output may be generated, including a representation of the speech waveform as a function of time, stored in digital or analog form, and/or a representation of speech based on temporal features, such as a windowed speech signal or features such as signal energy, mean, and/or zero-crossing rate, and/or a representation of speech based on its frequency or spectral contents (spectral features), for example, as a spectrogram, as cepstral coefficients and/or another representation derived from the speech frequencies, and/or a representation of vocal tract properties, such as linear prediction coefficients, or any combination temporal, spectral, or prosodic features.. Speech features further include any representation of prosodic features, such as the intonation, the fundamental frequency, i.e. the frequency of the vocal chords, and/or the signal energy.

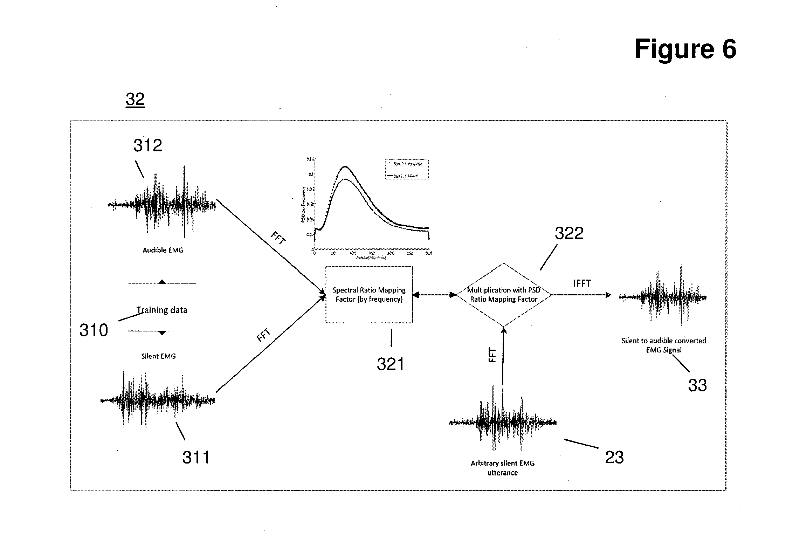

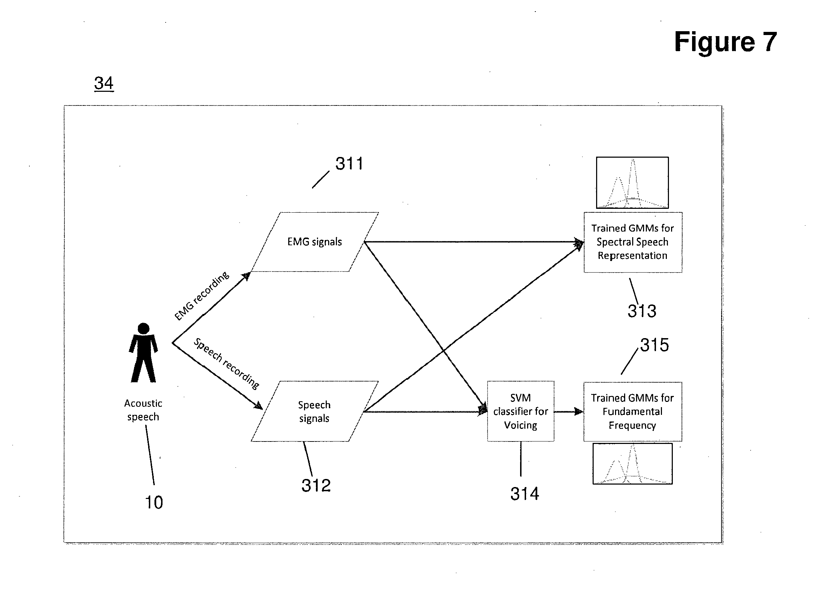

The representation of silent and/or audible speech is converted to speech features 33 corresponding to the previously captured and digitalized myoelectric signal 23. The silent audio conversion 30 is further described with reference to figures 6 to 8, wherein the silent mapping method 32 is described in greater detail with reference to figure 6 and the GMM conversion method 34 is described in greater detail with reference to figure 7.

During vocoding 40 of the captured and processed at least one myoelectric signal 21, the retrieved corresponding speech features 33 are transformed into corresponding audible speech 50 which is output by vocoding the resulting speech features 33 to generate at least one corresponding audible signal 42. The audible signal 42 generated from the speech features 33 is output using an output device 44 such as a loud speaker 50. In other words, at vocoding 40 the previously processed myoelectric signal resulting in speech features 33 is output as corresponding audible speech 50 by vocoding the speech features resulting from the processing of the captured myoelectric signal 21 during steps 20 and/or 30. Vocoding of the speech features of the processed myoelectric signal 33 results in a corresponding audible signal 42 which can be output as corresponding audible speech 50 using the output device 44. In one example, the speech features of the processed myoelectric signal 33 are synthesized into a speech waveform such that a human listener or a digital device (e.g. a digital phone, a computer, a robot, a personal digital device) receives a corresponding intelligible audible signal 42 through the output device 50.

The vocoding component output 40 may further provide useful user feedback to the speaker 10 and/or to other users/receivers in multiple modalities including audible (e.g. loudspeaker, ear plug), visible (e.g. computer screen, projector, Braille lines), and/or tactile (e.g. vibration sensor) feedback.

The intelligibility of the output audible speech 50 corresponding to the captured myoelectric signal 21 may depend on a quality of retrieved speech features during the processing steps 20 and/or 30 and/or on a quality of the vocoding 40. For example, when at least one myoelectric signal 21 results from silent articulatory activity of a speaker 10 (when the speaker 10 produces speech without uttering the speech loud or audible), a fundamental frequency needs to be estimated properly.

The fundamental frequency of audible speech is defined as the frequency with which the vocal chords vibrate. It is present only for voiced speech sounds, whereas for unvoiced speech sounds as well as for silent or whispered speech, a fundamental frequency is not defined. Fundamental frequency determines the intonation of speech and is therefore instrumental in capturing the intent of a spoken utterance, as well as making it sound intelligible and natural, and distinguishable in case a language uses tonal information for sound discrimination. In the context of the present application, fundamental frequency information comprises, for each frame of a speech signal, the information whether this frame is considered voiced or unvoiced, and in the first case, the fundamental frequency, measured in Hertz (e.g. 80 Hertz).

In silent and whispered speech, the fundamental frequency does not exist. Therefore, it is generated by the conversion process 30. Firstly, a computer-system based automated discrimination between different classes, herein referred to as "classifier" may be applied to discriminate voiced speech sounds from unvoiced speech sounds. This classifier takes any representation of the myoelectric signal, or parts thereof, as input, and outputs whether any speech frame is voiced or unvoiced. A possible classifier for this task is a Support Vector Machine (SVM). This voiced/unvoiced classification is followed by an estimator of the fundamental frequency on the voiced or audible speech features. This aspect is part of the GMM conversion method 34 described in further detail below with reference to figure 7. The estimated fundamental frequency is part of the speech features of the processed myoelectric signal 33 to be transformed during the vocoding 40.

In a preferred implementation of the system and method shown in figure 1, a speaker 10 may provide silent speech as input. If the speaker produces silent speech, he or she lacks the commonly available audible feedback from his/her own voice. In this case, valuable feedback generated by the audible output speech, or visible or tactile output 50 may be useful to be feed back to the speaker 10 by the system output 50. For example, multimodal feedback mechanisms in terms of proper audible, visible, and/or tactile forms which provide speakers 10 with feedback about their speech production quality. Audible feedback may be given in terms of audible speech and/or simpler forms of audible signals (e.g. a low pitch low volume tone if speech articulation is properly performed by the speaker, and a high tone of higher volume if speech articulation lacks quality. Visible feedback may be provided by life video of the performing speaker 10, pictures and/or video of corresponding articulatory movements, simulation of articulatory movement, and/or text-based and/or icon-based representation of the output. Tactile feedback may be provided by means of vibrating sensors.

By implementing the method and system comprising the above described steps 20, 30 and/or 40, silently produced speech of a speaker 10 is automatically and directly converted or transformed into corresponding audible signals 42 to be output as audible speech 50. In other words, the silently produced speech of a speaker 10 is automatically captured in terms of its corresponding myoelectric signals 21 and transformed into corresponding audible signals 42 which are output through an electronic output device 44 to be played by a loudspeaker 50.

In this way, applications ranging from human-human "silent speech communication" and silent human-machine interaction to providing replacement voices for users who can no longer speak due to age, weakness, disability and/or injury are enabled. Thus, speech communication is enabled when an audible signal is unavailable or corrupted or masked by surrounding noise of any kind. Such a situation may arise under several circumstances. For example, a user or speaker needs and/or wants to communicate to other persons and/or to digital devices, such as digital phones, computers, robots, personal digital assistants in public places where speaking aloud is inappropriate such as talking in a library, during a meeting, or in security scenarios, and/or making silent phone calls in public places. In another example, a user or speaker is at a place with a high ambient noise, where audible speech is masked by environmental noise, like airports, restaurants, or crowded places. Further, a user or speaker may need to transmit confidential and/or personal information, such as passwords, PINs, or proper names, when there are bystanders present. Normal, i.e. audible speech would breach the confidentiality of said communication. In yet another example, a user or speaker may not be able to utter audible speech due to speech-disabilities, accidents, general weakness, or permanent and temporary illness (e.g. laryngectomy patients). Those examples are advantageously addressed by the present application so that corresponding audible speech is automatically generated from myoelectric signals of a user or speaker in a very flexible and efficient manner.

**Figures 2 to 5** describe an exemplary implementation of the signal acquisition and processing 20 comprising a signal acquisition method 22 (see figure 2), a signal processing method 28 (see figure 5), a recording device 24 for recording myoelectric signals 21 (see figure 3), and a capturing device 26 for measuring, recording, and/or inputting myoelectric signals 21 (see figures 4A-C).

Basically, a movement of the human body results from contraction of the skeletal muscles. When a muscle fiber is activated by the central nervous system and performs a contraction, small electrical currents in form of ion flows are generated. These so-called action potentials move through the body tissue, encountering a resistance which generates an electrical field. The resulting potential differences can then be measured by surface electrodes attached noninvasively to the subject's (e.g. speaker 10) skin. Said recording method is referred to as electromyography recording so called myoelectric signals 21.

A single activation potential substantially lasts (about) 1 to 5 ms. During the period of a muscle contraction, each involved muscle fiber is constantly re-activated so that sequences of action potentials consisting of consecutive action potentials can be observed. On the skin surface, a superposition of many such action potential trains is measured, so that a myoelectric signal attains the properties of a pseudo-random stochastic process. For medical and physiological measurements, standard electromyography systems based on separate electrodes have been used for decades.

In one implementation, transformation of myoelectric signals 21 resulting from articulatory activity of a speaker 10 into corresponding audible speech is based on electromyography. Myoelectric signals 21, which result from articulatory activity of the speaker 10 representing speech activity of the speaker 10, relate to corresponding electric activity of the speaker's 10 muscles appearing during the articulatory activity. Using electromyography, the corresponding myoelectric signals produced by the speaker 10 during articulatory activity to, possibly silently, produce speech are capture using a capturing device 26 such as electrodes and/or arrays of electrodes 261, 262, 263 attached to the speaker's 10 body, face, throat, mouth, and/or neck. This relies on the observation that speech is also produced by activity of corresponding articulatory muscles of a speaker 10 so that resulting myoelectric signals 21 allow tracing back the corresponding audible speech intended to be output by the speaker 10. Further, since the myoelectric signals 21 captured using electromyography substantially emerge by muscle activity only, the corresponding speech can be automatically recognized and/or processed even if the speaker 10 speech output is produced silently, i.e. mouthed and/or articulated without any vocal effort.

**Figure 2** describes an exemplary implementation of the signal acquisition 22.

At least one myoelectric signal 21 is captured from a speaker 10 using a measuring device 26 including bipolar electrodes 261, unipolar electrodes 262, and/or reference electrodes 263. The bipolar and/or unipolar electrodes 261, 262 may be arranged and/or ordered as arrays and/or imprints, injections, and implants.

In an exemplary implementation, electrodes 261, 262, 263 with a diameter of 13 mm and a circular detection surface with a diameter of 4 mm can be used to capture myoelectric signals. Each electrode 261, 262, 263 may be separately attached to the skin of the speaker 10. In this case, the minimum inter-electrode distance may not be lower than 15 mm.

The electrodes 261, 262, 263 may be Ag-AgCl gelled surface electrodes. Ag-AgCl gelled surface electrodes measure electrical potential differences in the human body from the surface of the skin, they consist of a silver (chemical symbol: Ag) body on top of which a very thin layer of Silver chloride (AgCl) is applied. Between the Ag-AgCl body and the surface of the skin, electrolyte gel is applied to improve conductivity between the electrode and the skin surface. Ag-AgCl gelled surface electrodes are rather cheap and easy to obtain from a number of medical suppliers, they are frequently used in medical applications.. However, Ag-AgCl gelled surface electrodes might have one or more of the following limitations: Small changes in the electrode positioning between different recording sessions are difficult to detect and cannot be compensated. It is very difficult to identify the active muscles or motor units making the classification of the facial EMG signal challenging due to the multitude of superimposed signal sources which cannot be separated, and does not allow an in-depth study of the muscular activity.

Exemplary implementations of electrodes, arrays of electrodes, and/or implants 261, 262, 263 overcoming one or more of the above described limitations and which can be used as capturing device 26 are described below with reference to figures 4A to 4C.

A myoelectric signal 21 obtained from articulatory activity of a speaker 10 using a capturing device 26 may be represented as an analog signal. The myoelectric signal 21 of the user 10 can be processed by applying a noise filtering method 221 to the myoelectric signal. Different noise filtering methods exist such as analog or digital filters to versatile methods such as source separation, each designed to reduce the content of interfering signal from the information-bearing myoelectric signal. A differential amplification method 222 can be applied to the filtered myoelectric signal. Processing the myoelectric signal may further comprise a second stage filtering process 223.

The resulting processed myoelectric signal 21 is transmitted by a micro controller system 224 and then converted into a corresponding digital signal 23 by applying an analog/digital conversion method to the analog myoelectric signal 21.

Analog/digital conversion converts an analog signal, e.g. the electric current in a cable, to a digital signal, i.e. a sequence of numbers, which is stored and processed by a digital computer. Analog/digital conversion comprises two parts: quantization (discretization of signal values) and sampling (discretization of the time).

In an implementation, the quantization is preferably performed using a bit resolution between 15 and 20 bits (preferably 16 bit resolution). Sampling is preferably performed with a 1000 Hz sampling rate. Rather than using a 16 bit resolution in the quantization method, in one implementation a lower resolution can be used. Using a resolution lower than 16 bit, might however result in a worse output quality. In another implementation, rather than a 16 bit resolution, a higher resolution can be applied to the processed analog myoelectric signal 21. Using a resolution higher than 16 bit might however result in a larger data volume and thus larger storage requirements. In a preferred implementation, the sampling method is implemented by applying a 1000 Hz sampling rate to the previously processed myoelectric signal 21 to avoid aliasing. In another exemplary implementation, a sampling rate of 300 Hz is applied. Using such a rather low sampling rate (i.e. below 1000 Hz, e.g. about 300 Hz) may result in a critically low time resolution to capture the entire myoelectric signal 21. In yet another exemplary implementation, a sampling rate above 1000 Hz is applied to the processed myoelectric signal. Using a rather high sampling rate (i.e. above 1000 Hz) may require a larger data volume and thus larger storage requirements.

The at least one myoelectric signal 21 captured from the speaker 10 being processed by one or more or all of the above described methods 221, 222, 223, 224 and converted into a digital signal 23 by applying an analog/digital conversion method 208 to the myoelectric signal 21 can be further processed by the signal processing method 28 (described below with reference to figure 5).

**Figure 3** shows an exemplary recording device 24. The recording device 24 for recording electromyography-based speech data from a speaker 10 by means of myoelectric signals 21 comprises one or more analog inputs 241. The analog inputs 241 may comprise IN1 (for analog channels 1 to 6, as well as power and ground), IN2 (for analog channels 7 to 12, power and ground) and IN3 (for analog channels 13 to 16, two additional Marker channels which may allow to manually marking a particular event in the digital signal, power and ground). The analog inputs IN1, IN2, and IN may be connected with one or more capturing devices 26. The marker channel IN3 may be used for synchronizing myoelectric signals 21 with parallel recorded audio signals. The purpose of the recording device 24, together with the attached one or more capturing devices 26, is to perform the signal acquisition 22 step.

The recording device 24 can be connected to a computing environment 920, e.g. a laptop or a smart phone, via Bluetooth and/or a serial cable connection, for example by using a transmission rate of 230KBaud. Technical specifications of the recording device 24 may comprise an amplification factor of 1170, a 16 bits A/D converter, and/or a step size (resolution) of 0.033 microvolts per bit. The embedded A/D converter can use several sampling rates.

**Figures 4A and 4B** describe exemplary positioning of the capturing device 26 on a speaker's 10 skin for capturing one or more myoelectric signals resulting from articulatory activity of the speaker 10 representing speech. **Figure 4A** shows a capturing device 26 comprising one or more single electrodes arranged on the skin of a speaker 10. This is also referred to as a single-electrode system. **Figure 4B** shows a capturing device 26 comprising an array of electrodes arranged on the skin of a speaker 10. This is also referred to as an array-based electrode system.

Rather than constructing a capturing device 26 comprising single electrodes which might impose limits on the signal acquisition 22, a capturing is preferably constructed comprising an array of electrodes. Arrays of electrodes can be arranged on the skin surface of a speaker 10 whose articulatory activity representing speech is captured and processed to automatically output corresponding audible speech.

Using arrays of electrodes as a capturing device 26 increases the number of channels and thus the spatial resolution of myoelectric signals compared to usual separate-electrode systems. Since the positioning of the electrodes within an array of electrodes is known, it allows for techniques of independent component analysis and beamforming in order to discriminate signal sources (e.g. myoelectric signals) on a much finer granularity for tracing and classifying the complex muscular activity during speech production of a speaker 10.

Independent Component Analysis performs this task by extracting parts of the signal being statistically independent from each other, assuming that parts of the myoelectric signal originating from different muscles exhibit statistical independence. Beamforming does not require this precondition, however requires prior knowledge about the relative positioning of muscles or activity sources.

In other words, using arrays of electrodes during signal acquisition method 22 (see figure 1) allows for analyzing the spatial distribution of muscle activity, thus e.g. activity sources related to certain muscles may be detected for analyzing and/or mapping the spatial characteristics of muscle activity.

Myoelectric signals resulting from articulatory activity of a speaker 10 representing speech can be captured with a capturing device 26 built from different types of electrodes. The types of electrodes preferably used for the method shown in figure 1 are operable to convert an ionic current which is generated by the movement of a speaker's 10 muscle (representing the articulatory activity of the speaker 10) into an electronic current (representing one or more myoelectric signals), which then can be further processed. Exemplary implementations of the capturing device 26 comprise one or more of the following types of electrodes: single surface electrodes, miniature electrodes imprinted on the skin, injected into the skin or implanted directly under the skin, surface electrode arrays.

As shown in figures 4A and 4B, in one exemplary implementation of the signal acquisition method 22, a capturing device 26 captures myoelectric signals from muscles that are involved in the speech articulation process of a speaker 10 (the articulatory activity of the speaker 10), which might be the levator angulis oris, the zygomaticus major, the platysma, the anterior belly of the digastric, and/or the tongue. However, due to the fact that myoelectric signals might be captured at the surface of the speaker's 10 skin, some of the captured myoelectric signals may consist of a superposition of active muscle fibers in the proximity of the recording electrode. To filter out such artifacts, a captured myoelectric signal may be further processed in the acquisition and processing 20.

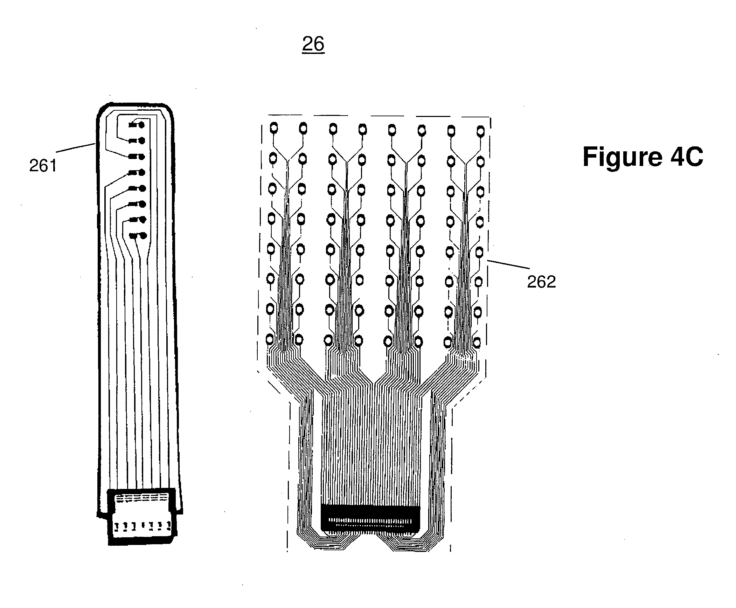

**Figure 4C** shows exemplary implementations and/or constructions of a capturing device 26 in terms of arrays of electrodes 261, 262, also referred to as array-based electrode systems 261, 262. One or more myoelectric signals captured using the capturing device 26 can be recorded using a recording device 24 (e.g. the recording device 24 shown in figure 3).

Two different types of electrode arrays 261, 262 are shown in figure 4C. A first exemplary array of electrodes 261 is a semi-disposable adhesive array of electrodes 261, wherein the electrodes in the array 261 are arranged with a 5mm inter electrode distance. A second exemplary array of electrodes 262 is a semi-disposable adhesive array of electrodes 262, wherein 64 electrodes are arranged in the array 262 and wherein the electrodes are arranged with a 10mm inter electrode distance.

The electrodes in the arrays 261, 262 can be used with unipolar derivation or with bipolar derivation. Unipolar derivation means that potential differences between the array channels and one or more reference electrodes are measured, a reference electrode may be placed e.g. in the back of the neck of a user 10. In a bipolar derivation the potential difference between two adjacent channels is measured.

**Figure 5** shows an exemplary implementation of the signal processing method 28 which is part of the signal acquisition and processing 20 shown in figure 1. The signal processing method 28 may be implemented subsequent to the signal acquisition method 22 as described with reference to figure 2. Further, one, some or all of the shown steps of the signal processing method 28 may be preformed prior, during, and/or after a silent mapping method 32.

The myoelectric signal 21 being captured, filtered and/or amplified by the acquisition method 22 as described above with reference to figure 2, is converted from said analog myoelectric signal 21 to a corresponding digital signal 23 by applying an analog/digital conversion method consisting of sampling and quantization as described with reference to figure 2. In a preferred implementation, the quantization uses 16 bit resolution, and the sampling applying a 1000 Hz sampling rate. The digital signal 23 is a digitalized representation of at least one myoelectric signal 21 captured from a speaker 10 as described above with reference to figures 1 and 2 and is also referred to herein as a digitalized myoelectric signal 23.

During the signal processing method 28, the received digital signal 23 representing the initially captured myoelectric signal 21 is digitally processed by applying subsequently and/or concurrently an artifacts removal and filtering method 281, a feature extraction method 282, and/or a feature dimensionality reduction method 283.

The artifacts removal and filtering method 281 may comprise source separation or localization methods such as Independent Component Analysis, Beamforming, and/or related methods.

As already mentioned the artifacts removal and filtering method 281 removes artifacts from the received digitalized myoelectric signal 23. Artifacts which deteriorate the signal quality may arise from properties of the human body of the speaker and/or from the applied technology. Examples of such artifacts may comprise low-frequency waves due to sweating, 50Hz or 60Hz frequency injection from surrounding power sources (power line noise), and/or signal deviations due to movement of the electrode connector cables.

In an exemplary implementation of the artifacts removal and filtering method 281 to remove such kinds of artifacts, the digitalized myoelectric signal 23 may be filtered with a high-pass filter with a cutoff frequency preferably ranging between 1 Hz and 10 Hz. Subsequently and/or alternatively, a 50 Hz or 60 Hz notch filter may be applied to said signal 23 to cut out power line noise. This might however lower a quality of the originally captured myoelectric signal. Further and/or alternatively, the digital signal 23 can be filtered with an analog low-pass filter with a cutoff frequency according to the Nyquist rate of 500 Hz to avoid aliasing.

In a preferred implementation of the artifacts removal and filtering method 281, artifacts may be removed from the digitalized myoelectric signal 23 in an electrode array based system by applying Blind Source Separation (BSS). The BSS applies a linear transformation on the digitalized myoelectric signal 23 to split said signal 23 into components which satisfy a certain (given) criterion. In this component representation, myoelectric components are separated from a wide range of artifact components. A known heuristical classification method may be implemented which is based on certain properties of a component, e.g. autocorrelation or frequency spectrum, to distinguish an artifact component from a myoelectric component in the digitalized myoelectric signal 23. The detected artifact components may be removed from the digitalized myoelectric signal 23. Speech features are extracted from said filtered myoelectric signal, i.e. the digitalized myoelectric signal 23 with removed artifact components. An example of a BSS algorithm is the Independent Component Analysis (ICA). Other known source separation algorithms are also feasible to implement the BSS algorithm. The BSS algorithm used to implement the artifacts removal and filtering method 281 may perform steps of isolating and/or removing artifacts from the myoelectric signal and/or of separating the activity of different facial muscles.

In an exemplary implementation in case a few properties of the captured myoelectric signal are assumed, the artifacts removal and filtering method 281 may in addition and/or alternatively to the BSS algorithm implement a beamforming step. During beamforming each channel of a capturing device used to capture the myoelectric signal is filtered separately in order to enhance a signal originating from a specific muscle or muscle group and/or to attenuate interfering signals either from different muscles or artifact-caused. Known beamforming algorithms which can be implemented with the artifacts removal and filtering method 281 may comprise classical algorithms such as delay-and-sum beamforming, adaptive beamforming, and/or versatile approaches based on the objective of optimizing the correlation, and/or another similarity measure between an output of the beamforming and the target audible signal corresponding to the captured myoelectric signal.

Relevant features are extracted from the raw myoelectric signal, which represent the muscular or articulatory activity of the speaker. These features may include a windowed representation of the myoelectric signal, which is used to estimate the timing of muscular activities. Furthermore, from the raw or windowed myoelectric signal, features may be computed based on the time domain *(temporal features),* like signal energy, mean, zero-crossing rate, etc., or based on the frequency domain *(spectral features),* like short-time spectral, cepstral, or filtered features, or based on other feature extraction methods, like wavelet features. Any such features may be combined to optimally represent the myoelectric signal, yielding e.g. spectro-temporal features. In a preferred implementation, the feature extraction method 282 applied to the digitalized myoelectric signal 23 is based on time-domain features.

The feature dimensionality reduction method 283 has the purpose of reducing the high number of dimensions of the extracted features of the myoelectric signal, so that further processing becomes possible. The feature dimensionality reduction method 283 may apply a linear discriminant analysis (LDA) on the digitalized myoelectric signal 23, or the extracted features of the digitalized myoelectric signal 23 in order to reduce the high dimensionality of the multi-channel combination of speech features obtained during the feature extraction 282 of said signal 23. Furthermore, principal component analysis, or related methods, may be applied before or after the LDA step as an additional feature reduction step.

In an exemplary implementation of the feature extraction method 282, for each given feature f of the digitalized myoelectric signal 23, the feature's frame-based time-domain mean $\bar{f}$, its frame-based power $P_f$, and/or its frame-based zero-crossing rate $Z_f$ are defined. $S(f,n)$ defines the stacking of adjacent frames of the feature f in the size of *2n+1* (-n to n) frames. The construction of adjacent feature vectors is expected to capture more complex information than simple first order or second order derivative information. For a myoelectric signal (or its digitalized representation 23, respectively) with normalized mean *x[n],* the nine-point double-averaged signal *w[n]* is defined as:

$$w[n] = \frac{1}{9} \sum_{k=-4}^{4} v[n+k]$$

where

$$w[n] = \frac{1}{9} \sum_{k=-4}^{4} v[n+k]$$

The high-frequency signal is *p[n]= x[n] - w[n],* and the rectified high-frequency signal is *r[n] = |p[n]|*.

In an exemplary implementation using a single-electrode system (e.g. electrodes 263 in figure 2) for capturing myoelectric signals, a Feature Set called *TD15* with *TD*15 = S/*f*2,15),where *f*2 = $[\overline{w},P_w,P_r,Z_p,\overline{r}]$. is implemented.

in an exemplary implementation using an array-based electrode system (e.g. the arrays of electrodes 261, 262 shown in figure 4C), the stacking delta is reduced to 5 or 10 frames in order to avoid dimensionality artifacts. Frame size and frame shift are preferably set to 27 ms and 10 ms, respectively.

In both of the above described implementations of the feature extraction method 282, preferably a Linear Discriminant Analysis (LDA) is applied on the TD15 feature to generate the final feature vector with 32 coefficients.

Figures 6 to 8 show exemplary methods for implementing silent/audio conversion 30 as shown in figure 1. Silent/audio conversion 30 comprises a silent mapping method 32 as described below with reference to figure 6 and/or a Gaussian mixture model (GMM) conversion method 34 as described below with reference to figure 7.

In general, recorded, processed and digitalized myoelectric signals 23 are synthesized into audible myoelectric signals 33 representing audible speech. The digitalized myoelectric signals 23 result from digitalizing myoelectric signals 21 captured from a speaker's body, face, throat, mouth, neck, etc. are captured to obtain articulatory data of articulatory activity of the speaker representing speech. The captured myoelectric signals 21 may be digitally processed as described above with reference to figures 2 and 5. Said digitalized myoelectric signals 23 are converted into corresponding audible myoelectric signals 33 representing audible speech which corresponds to the articulatory activity of the speaker 10 represented by the captured myoelectric signals 21.

In a preferred implementation, captured, processed and digitalized myoelectric signals 23 are converted into the corresponding audible myoelectric signals 33 for outputting the corresponding audible speech by implementing, preferably within the silent/audio conversion component 30, a combination of two methods 32 and 34: a silent mapping method 32 preferably followed by a Gaussian mixture model (GMM) conversion method 34. Converting myoelectric signals 23 into corresponding audible myoelectric signals 33 using said two methods 32 and 34 enables (silent) speech communication of a user or speaker when an acoustic signal (of the speaker) is unavailable.

The silent mapping method 32 and the GMM conversion method 34 may be both implemented using a same enrollment phase also referred to as a training phase. During the training phase, parameters of the silent audio conversion 30 are estimated and/or adapted to a current speaker 10. For and/or during the training phase, audible signals 312 are captured in addition to myoelectric signals 311 from the speaker 10. Audible signals 312 and myoelectric signals 311 captured from the speaker 10 during the training phase are referred to as training data 310. In a preferred implementation, the training phase is performed immediately before using the myoelectric signal to audible speech conversion according to the method and system describe herein (e.g. figure 1). In another implementation, the myoelectric signal to audible speech conversion according to the methods and systems describe herein (e.g. figure 1) may be used without the need for a training phase.

Audible signals 312 may be captured during the training phase using a common kind of microphone such as a standard headset microphone. Preferably, an audible signal 312 is quantized with a 16 bit resolution and sampled with a sampling rate of 16 kHz. In other implementations, other quantization and/or sampling rates are used such as when using a mobile appliance with reduced capabilities.

For the training phase, mel-cepstral features are additionally computed on audible signals 312, where the exact speech features to be used may vary among standard speech representations, including mel-frequency cepstral coefficients, linear prediction coefficients, etc.

Mel-frequency Cepstral Coefficients (MFCC) are a representation of the short-time power spectrum of a sound. This representation is based on a linear cosine transform of the log power spectrum on a non-linear mel-scale of frequency (see for example wikipedia). The mel-scale mimics the performance of the human ear. Furthermore, since the shape of the human vocal tract is manifested in the envelope of the short-time power spectrum, MFCCs are a compact and accurate representation of human sounds which are produced and perceived by humans. MFCCs are widely used in speech processing. After their introduction by Davis and Mermelstein in the 1980's they have been state-of-the-art ever since. Prior to MFCCs, Linear Prediction Coefficients (LPCs) and Linear Prediction Cepstral Coefficients (LPCCs) were the main features.

Figure 6 shows an exemplary implementation of the silent mapping method 32 as used within the implementation of the silent/audio conversion 30.

The silent mapping method 32 as shown in figure 6 compensates for differences between the EMG signals of audibly and silently spoken speech. This may arise from observations that the magnitude of a myoelectric signal 311 of silently uttered speech of a speaker is significantly lower than a myoelectric signal of corresponding audible uttered speech of the speaker 10 with the same speech content.

The silent mapping method 32 may be performed before, during, and/or after the feature extraction method 282 and subsequent feature dimensionality reduction method 283 described above with reference to figure 5. In a preferred implementation, the silent mapping method 32 is applied to a digitalized myoelectric signal 23 after the artifact removal method 281, but before the feature extraction method 282 and before a subsequent feature dimensionality reduction method 283 are applied to a myoelectric signal 23.

Advantageously, since the silent mapping method 32 compensates differences between audibly and silently spoken speech (i.e. between two myoelectric signals, one resulting from producing audible speech when uttering a sentence, the other resulting from producing silent speech when uttering the same sentence by a speaker 10), the speaker 10 may perform seamless switches between audibly and/or silently uttering speech in the middle of a spoken utterance without significant loss of performance. In other words, the speaker may speak (or utter speech) either silent or audible as well as switching back and forth without the need of changing the feature extraction and/or parameter settings as performed during the signal processing method 28 as shown with reference to figure 5.

The silent mapping method may require a training phase before it can be applied, in which the parameters of the mapping are estimated, as follows: During the training phase for the silent/audio conversion 20, a small set of recordings of speech uttered by users or speakers is performed, wherein each sentence or phrase uttered by a speaker 10 is recorded twice: once, the sentence or phrase is uttered audible or loud by a speaker 10 to produce an audible signal 312 from audible speech and once said sentence or phrase is uttered silent (e.g. silently mouthed) by said speaker 10 to produce a myoelectric signal 311 from silent speech. During the training phase, the silent mapping method 32 transforms the set of said training data 310 into the frequency domain (a popular way of characterizing speech is in terms of a signal or acoustic waveform, i.e. amplitude over time, the so-called time domain; for many types of signals such as speech it is favorable to represent the speech signal as frequency over time, the so-called frequency domain; to transform between time domain and frequency domain, the well-known Fourier Transform can be applied; typically, several consecutive frequencies may be pooled into frequency bins to be represented by the same value) i.e. performs a frequency transformation) and computes the energy per frequency bin averaged over all recordings of audible signals 312 and corresponding myoelectric signals 311 of the speaker 10, 321. These energy values form the parameters which are required for later applying the silent mapping method.

In the silent mapping method 32, a frequency domain transformation may be performed implementing various different standard methods for transforming frequency domains. In one preferred implementation, the frequency domain transformation is performed using a so-called Power Spectral Density (PSD) method of at least one digitalized myoelectric signal 23 captured from a speaker 10. The PSD of a random process can be expressed as the Fourier transform of its autocorrelation function (see for example Huang et al., equation 5.261). The PDD method thus relates to the Fourier transform of a signal but is smoother than a numerical estimate of the Fourier transform. Different known PSD methods and/or known smoothing mechanisms may be applied for the Fourier transform.

In a preferred implementation, the PSD method is implemented by the known Welch method. The Welch's method for the PSD method for smoothing the Fourier transformation may be implemented as follows:

- The input signal 311, 312, 23 is divided into an integer number of segments, preferably with a 30 samples window length with 67% overlap.

- Each segment is windowed with a Hamming window to reduce spectral distortion. The generalized Hamming window is defined as:

$$h[n] = \begin{cases} (1 - \alpha) - \alpha \cos\left(\dfrac{2\pi n}{N}\right) & 0 \le n < N \\ 0 \ otherwise \end{cases} \qquad x_t = [x_t(1), \dots, x_t(d_x)]^\top$$

If $\alpha = 0.46$ this window is known as the Hamming window.

- On each segment, the Fast Fourier Transform (FFT) is computed, yielding a Fourier representation of the signal 311, 312, 23.

During the training phase, the PSD method may be computed on a per-utterance and per-channel basis and then averaged over the recorded signals 311, 312 to compute an average value of the spectral information of the recorded audible signals 312 and the recorded myoelectric signals 311. Computing an average on a per-channel basis results in a more accurate estimate rather than computing the average over all channels.

A ratio of said averaged spectral content can be defined as the ratio between the spectral energy of the recorded signals 311, 312 by frequency bin resulting in a spectral ratio mapping factor 321.

When the training phase is terminated, at least one digitalized myoelectric signal 23 captured from a speaker 10 representing a silently (mouthed) uttered speech is transformed into the frequency domain computed during the trainings phase. For each channel of the myoelectric signal 23, a frequency of the myoelectric signal 23 is multiplied with its spectral ratio mapping factor, 322 and the result of said multiplication is transformed back into the time domain to obtain the corresponding audible myoelectric signal 33. In one exemplary implementation, to compute such an invertible frequency domain transformation standard, FFT (Fast Fourier Transform) and inverse FFT (IFFT) are implemented.

The previously described implementation of a silent mapping method 32 is a preferred implementation of alleviating the differences between myoelectric signals resulting from silent speech and audible speech, respectively. The silent mapping method 32 may be modified in several ways. For example, mapping ratios between myoelectric signals corresponding to audible speech 312 and myoelectric signals corresponding to audible speech 311 may not be computed on frequency bins, but on preprocessed features resulting from the signal processing method 28. In another example, the multiplication step 322 of the silent mapping method 32 may be replaced by a more complex mapping algorithm. For example, rather than applying the same spectral ratio mapping to each channel of the myoelectric signal 23, as described above, one could vary the ratio depending on a linguistic classification the myoelectric signal belongs to, such as vowels versus consonants, or one could apply different ratios to different channels depending on the position in the face, filter property or frequency range of the corresponding electrodes.

Figure 7 shows an exemplary implementation of a Gaussian mixture model (GMM) conversion method 34 as used within the implementation of the silent/audio conversion 30 is shown.

In the GMM conversion method 34, a Gaussian mixture model (GMM) of joint probability densities is trained based on synchronously recorded and processed audible signals 312 and myoelectric signals 311. The myoelectric signals 311 may be captured from at least one speaker 10 according a method 22 as shown in figure 2.

Audible signals 312 and myoelectric signals 311 are recorded from a plurality of speakers 10 in several recording sessions in a parallel setup. In an implementation, a specific protocol and fixed sets of sentences to be uttered audible and silently are created to ensure comparability across the speakers 10. Myoelectric signals 311 and audible signals 312 are separately digitally processed (e.g. using the method shown in figure 2) and features are extracted (e.g. using the method shown in figure 5) separately for each kind of the signals 311, 312. Joint feature vectors of the features extracted from both kinds of signals 311 and 312 are generated by stacking the features extracted from the myoelectric signals 311 and the features extracted from the audible signals 312. Said joint feature vectors are used to train a GMM to predict the spectral representation of speech for a spectral speech representation subsystem 313. In a preferred implementation the differences between myoelectric signals resulting from silent and audible speech are alleviated using the above described silent mapping method 32. However, the described GMM conversion method 34 could also be applied on myoelectric signals prior to silent mapping.

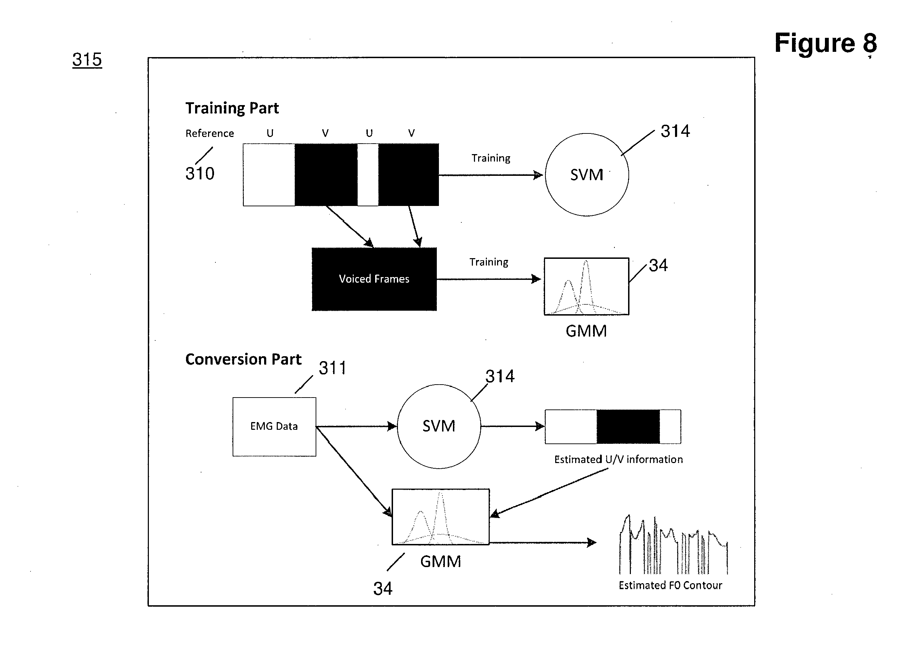

The fundamental frequency -- often referred to as "fundamental" and usually abbreviated as $f_0$ -- is defined as the lowest frequency of a periodic waveform. For a speech waveform in the vocal tract one finds $f_0 = v/2L$ where $v$ is the wave velocity of the sound wave (v=343.2 m/s at 20° C) and L is the length of the human vocal tract. The fundamental frequency, often referred to simply as the fundamental, is defined as the lowest frequency of a periodic waveform. The fundamental frequency is extracted from the audible signal 312. Speech prosody is typically described in terms of acoustics as variation in syllable length, loudness and pitch. Thus $f_0$ is one parameter typically used to describe speech prosody. Said speech prosody may be used to train a prosody subsystem 315 which can generate $f_0$ exclusively based on a myoelectric signal 311. Said prosody subsystem 315 and the related method are described in greater detail below with reference to figure 8.

Spectral speech features such as the well-known Mel-Frequency Cepstral Coefficients which represent the short-term power spectrum of the signal of a sound (MFCC) or derivatives of any kind of cepstral coefficients are generated and/or extracted from the myoelectric signals 311 and/or the audible signals 312 in the spectral speech representation subsystem 313. The spectral speech features can be extended by prosodic data from the prosody subsystem 315. Said extended features can be fed to the vocoding component 40 for generating an audible signal 42 from a captured myoelectric signal 21 of a speaker 10.

In the GMM conversion method 34, a GMM describes the relationship between input signals 311, 312 and the corresponding audible speech. When the GMM conversion method 34 is trained, the method 34 may accept arbitrary input digitalized and processed myoelectric signals 23 and may be not bound to a specific feature representation myoelectric signals and/or audible signals. Therefore, a feature extraction method different from the exemplarily described one above with reference to figure 5 may be implemented with the GMM conversion method 34. While the GMM conversion method maps the given feature representation frame-by-frame, other methods such as unit-based mapping could be applied as well. In the unit-based mapping method several frames are merged into larger-span units. The mapping then maps the entire source unit to a target unit by finding the closest match among the available target units. This mapping may be implemented as a GMM-based mapping or a pattern-matching method finding the closest match by a suitable distance measure between units.

This GMM-based conversion method 34 may comprise a training phase and a conversion phase.

For the training phase, source and target data are defined for the GMM conversion method 34. The source data may comprise myoelectric features extracted from myoelectric signals 311 using an extraction method (e.g. the signal processing method 28 as described with reference to figure 5), and the target data may comprise a spectral representation of audible signals 312 such as mel frequency cepstral coefficients. The myoelectric features may be stored in terms of myoelectric feature vectors and the spectral representation of the audible signals 312 (also referred to as source feature vector) may be stored as spectral target vectors (also referred to as target feature vector). Said two vectors may be concatenated in the training phase of the GMM conversion method 34.

A static source feature vector and a target feature vector may be defined at frame t as

$$x_t = [x_t(1), ..., x_t(d_x)]^\tau \text{ and } y_t = [y_t(1), ..., y_t(d_y)]^\tau .$$

$d_x$ and dy denote the dimension of source $x_t$ and target $y_t$, respectively. $\tau$ denotes transposition. After preparing training data of audible signals 312 and myoelectric signals 311, a GMM is trained to describe the joint probability density of the source feature vector and the target feature vector as follows:

$$P(X_t, Y_t \mid \lambda) = \sum w_m N\left(\left[X_t^T, Y_t^T\right]^T \mid \mu_m^{(X,Y)}, \Sigma_m^{(X,Y)}\right)$$

$$\mu_m^{(X,Y)} = \begin{bmatrix} \mu_m^{(X)} \\ \mu_m^{(Y)} \end{bmatrix}, \Sigma_m^{(X,Y)} = \begin{bmatrix} \Sigma_m^{(XX)} & \Sigma_m^{(XY)} \\ \Sigma_m^{(YX)} & \Sigma_m^{(YY)} \end{bmatrix}$$

where $N(; \mu, \Sigma)$ denotes the Gaussian distribution with a mean vector $\mu$ and a covariance matrix Z, m denotes a mixture component index, and M denotes the total number of the mixture components. The parameter set of the GMM is denoted by $\lambda$, which consists of weights $w_m$, mean vectors $\mu_m$(X,Y ) and full covariance matrices $\Sigma_m$(X,Y ) for individual mixture components. $\mu_m$(X) and $\mu_m$(Y) represent the mean vectors of the mth mixture component for the source features and the target features, respectively. $\Sigma_m$(XX) and $\Sigma_m$(YY) represent the covariance matrices and $\Sigma_m$(XY) and $\Sigma_M$(YX) represent the cross-covariance matrices of the mth mixture component for the source features and the target features, respectively.

The conversion process itself is based on the manner of minimum mean square error as follows:

$$\hat{Y}_t \quad = \quad \sum_{m=1}^{M} P\left(m|\boldsymbol{x}_t, \lambda\right) \boldsymbol{E}_{m,t}^{(Y)},$$

$$P\left(m|\boldsymbol{x}_t, \lambda\right) \quad = \quad \frac{w_m \mathcal{N}\left(\boldsymbol{X}_t; \mu_m^{(X)}, \Sigma_m^{(XX)}\right)}{\sum_{n=1}^{M} w_n \mathcal{N}\left(\boldsymbol{X}_t; \mu_n^{(X)}, \Sigma_n^{(XX)}\right)},$$

$$\boldsymbol{E}_{m,t}^{(Y)} \quad = \quad \mu_m^{(Y)} + \Sigma_m^{(YX)} \Sigma_m^{(XX)^{-1}} \left(\boldsymbol{X}_t - \mu_m^{(X)}\right),$$

where $\hat{Y}_t$ is the target joint feature vector consisting of the estimated static feature vector and dynamic feature vector $\Delta y_t^T$ at frame t. This vector may be specified as the estimated Mel Cepstral feature vector.

To further improve the GMM training the audible signal 312 may be used in a traditional known speech recognition system to produce phone alignments. Based on these phone alignments a more specialized set of GMMs could be trained.

When the training is terminated, the trained GMM may be used as follows: At least one digitalized a processed myoelectric signal 23 (e.g. using the methods shown in figures 2, 5, and/or 6) is fed into the silent/audio conversion 30. Using the GMM conversion method 34, a spectral representation of speech is generated from the myoelectric signal 23 using the spectral speech representation subsystem 313, and an estimate for the fundamental frequency of said spectral representation of speech is computed using the prosody subsystem 315. The resulting audible myoelectric signal is then passed on to the vocoding component 40, which creates a waveform representation of the speech, i.e. a corresponding audible signal 42, which can be directly played to a receiving user using an output device 44.

**Figure 8** shows an exemplary implementation of a prosody subsystem 315 which can be implemented within the silent/audio conversion component 30.

The prosodic subsystem 315 generates prosodic speech features, so-called fundamental frequency $f_0$. The generated prosodic speech features can be combined with the spectral speech features generated by the spectral speech representation subsystem 313 and fed to the vocoding component 40 to generate the corresponding audible speech 42 from the captured myoelectric signal 304 of a user 10.

The GMM conversion method 34 used for the spectral speech representation subsystem 313 is also used for the prosodic subsystem 315 (see figure 7). Input data to the prosodic subsystem 315 comprises myoelectric features generated from a myoelectric signal 21 and target data comprises the prosodic features extracted from the parallel recorded audible signal 312 such as the fundamental frequency values which could be used by itself of with additional coefficients describing the differential and acceleration of $f_0$ also known as differential and acceleration coefficients. The MFCC feature vector describes only the power spectral envelope of a single frame, but it se, also known as delta-coefficients.

In an exemplary implementation, a Support Vector Machine (SVM) 314 for discriminating frames from audible signals 312 and myoelectric signals 311 may be implemented. After the SVM discriminated voiced from unvoiced frames, $f_0$ values may be generated out of the voiced frames by the statistical GMM-based conversion method 34. For the unvoiced frames no $f_0$ is generated and thus the unvoiced frames are kept unaltered.

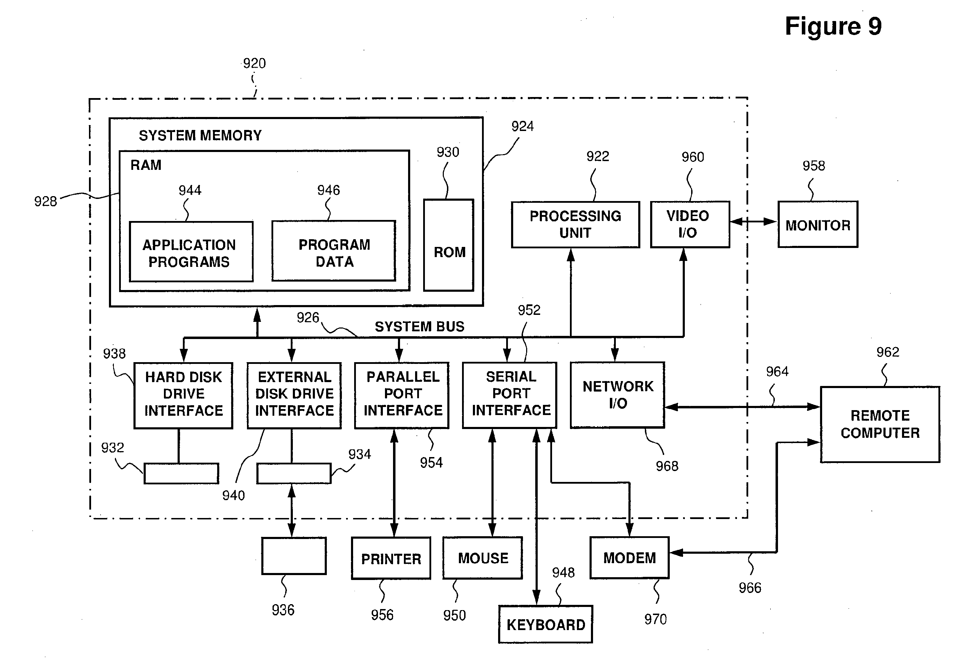

**Figure 9** shows an exemplary system for implementing the invention including a general purpose computing device in the form of a conventional computing environment 920 (e.g. a personal computer). The conventional computing environment includes a processing unit 922, a system memory 924, and a system bus 926. The system bus couples various system components including the system memory 924 to the processing unit 922. The processing unit 922 may perform arithmetic, logic and/or control operations by accessing the system memory 924. The system memory 924 may store information and/or instructions for use in combination with the processing unit 922. The system memory 924 may include volatile and nonvolatile memory, such as a random access memory (RAM) 928 and a read only memory (ROM) 930. A basic input/output system (BIOS) containing the basic routines that helps to transfer information between elements within the personal computer 920, such as during start-up, may be stored in the ROM 930. The system bus 926 may be any of several types of bus structures including a memory bus or memory controller, a peripheral bus, and a local bus using any of a variety of bus architectures.

The personal computer 920 may further include a hard disk drive 932 for reading from and writing to a hard disk (not shown), and an external disk drive 934 for reading from or writing to a removable disk 936. The removable disk may be a magnetic disk for a magnetic disk driver or an optical disk such as a CD ROM for an optical disk drive. The hard disk drive 932 and the external disk drive 934 are connected to the system bus 926 by a hard disk drive interface 938 and an external disk drive interface 940, respectively. The drives and their associated computer-readable media provide nonvolatile storage of computer readable instructions, data structures, program modules and other data for the personal computer 920. The data structures may include relevant data for the implementation of the method for automatic transformation of myoelectric signals into audible speech, as described above. The relevant data may be organized in a database, for example a relational database management system or an object-oriented database management system.

Although the exemplary environment described herein employs a hard disk (not shown) and an external disk 936, it should be appreciated by those skilled in the art that other types of computer readable media which can store data that is accessible by a computer, such as magnetic cassettes, flash memory cards, digital video disks, random access memories, read only memories, and the like, may also be used in the exemplary operating environment.

A number of program modules may be stored on the hard disk, external disk 936, ROM 930 or RAM 928, including an operating system (not shown), one or more application programs 944, other program modules (not shown), and program data 946. The application programs may include at least a part of the functionality as depicted in figures 1 to 8

A user may enter commands and information, as discussed below, into the personal computer 920 through input devices such as keyboard 948 and mouse 950. Other input devices (not shown) may include a microphone (or other sensors), joystick, game pad, scanner, or the like. These and other input devices may be connected to the processing unit 922 through a serial port interface 952 that is coupled to the system bus 926, or may be collected by other interfaces, such as a parallel port interface 954, game port or a universal serial bus (USB). Further, information may be printed using printer 956. The printer 956 and other parallel input/output devices may be connected to the processing unit 922 through parallel port interface 954. A monitor 958 or other type of display device is also connected to the system bus 926 via an interface, such as a video input/output 960. In addition to the monitor, computing environment 920 may include other peripheral output devices (not shown), such as speakers or other audible output.

The computing environment 920 may communicate with other electronic devices such as a computer, telephone (wired or wireless), personal digital assistant, television, or the like. To communicate, the computer environment 920 may operate in a networked environment using connections to one or more electronic devices. Fig.9 depicts the computer environment networked with remote computer 962. The remote computer 962 may be another computing environment such as a server, a router, a network PC, a peer device or other common network node, and may include many or all of the elements described above relative to the computing environment 920. The logical connections depicted in Fig.9 include a local area network (LAN) 964 and a wide area network (WAN) 966. Such networking environments are commonplace in offices, enterprise-wide computer networks, intranets and the Internet and may particularly be encrypted.

When used in a LAN networking environment, the computing environment 920 may be connected to the LAN 964 through a network I/O 968. When used in a WAN networking environment, the computing environment 920 may include a modem 970 or other means for establishing communications over the WAN 966. The modem 970, which may be internal or external to computing environment 920, is connected to the system bus 926 via the serial port interface 952. In a networked environment, program modules depicted relative to the computing environment 920, or portions thereof, may be stored in a remote memory storage device resident on or accessible to remote computer 962. Furthermore other data relevant to the method for optimization of evaluation of a policy (described above) may be resident on or accessible via the remote computer 962. It will be appreciated that the network connections shown are exemplary and other means of establishing a communications link between the electronic devices may be used.

The above-described computing system is only one example of the type of computing system that may be used to implement the method for automatic transformation of myoelectric signals into audible speech or into speech output corresponding to audible speech.

**List of Reference Numerals**

|    |                                   |
|----|-----------------------------------|
| 10 | speaker (also referred to as user) |
| 20 | signal acquisition and processing |
| 21 | myoelectric signal                |
| 22 | signal acquisition                |
| 23 | digitalized myoelectric signal    |
| 24 | recording device                  |
| 26 | capturing device                  |
| 28 | signal processing                 |
| 30 | silent/audio conversion           |
| 32 | silent mapping method             |

| 33 | speech features |
|---|---|
| 34 | GMM conversion method |
| 40 | vocoding |
| 42 | audible signal |
| 44 | output device |
| 50 | audible speech |
| 208 | analog/digital conversion method |
| 221 | noise filtering method |
| 222 | differential amplification method |
| 223 | second stage filtering process |
| 224 | micro controller system |
| 241 | analog inputs |
| 261, 262 | arrays of electrodes (array-based electrode system) |
| 263 | electrodes (single electrode system) |
| 281 | artifacts removal and filtering method |
| 282 | feature extraction method |
| 283 | feature dimensionality reduction method |
| 311 | audible signal |
| 312 | myoelectric signal |
| 313 | spectral feature representation subsystem |
| 314 | SVM |
| 315 | prosody subsystem |
| 321 | ratio mapping |
| 322 | multiplication with PSD |
| 920 | conventional computing environment |
| 922 | processing unit |
| 924 | system memory |
| 926 | system bus |
| 928 | random access memory (RAM) |
| 930 | read only memory (ROM) |
| 932 | hard disk drive |
| 934 | external disk drive |
| 936 | removable disk |
| 938 | hard disk drive interface |
| 940 | external disk drive interface |
| 944 | one or more application programs |
| 946 | program data |
| 948 | keyboard |
| 950 | mouse |
| 952 | serial port interface |
| 954 | parallel port interface |
| 956 | printer |
| 958 | monitor |
| 960 | video input/output |
| 962 | remote computer |
| 964 | local area network (LAN) |
| 966 | wide area network (WAN) |
| 968 | network I/O |
| 970 | a modem |

**Claims**

1. Computer-implemented method for automatic transformation of myoelectric signals (21, 23) into speech output (42) corresponding to audible speech (50), the method comprising:

capturing, from a human speaker (10), at least one myoelectric signal (21) representing speech;
converting at least part of the myoelectric signal (21) to one or more speech features (33); and

vocoding the speech features (33) to generate and output the speech output (42) corresponding to the myoelectric signal (21).

2. The method according to claim 1, wherein a representation of the one or more speech features is a spectral representation of speech comprising spectral features, temporal features, and/or spectro-temporal features.

3. The method according to claim 2, further comprising:

computing an estimate for a fundamental frequency of the spectral representation of speech.

4. The method according to any one of the preceding claims, wherein joint feature vectors resulting from a prior training phase on myoelectric signals (311) and corresponding audible signals (312) are used to predict the representation of speech for the myoelectric signal (21).

5. The method according to any one of the preceding claims, wherein the at least one myoelectric signal (21) is captured from the speaker's (10) head, throat, face, mouth, chest, and/or neck using an array-based electrode system (26, 261, 262).

6. The method according to any one of the preceding claims, wherein the speech is produced by the speaker (10) comprising normally articulated speech, whispered speech, murmured speech, speech that is barely or not audible to a bystander, and/or silently mouthed speech.

7. The method according to any one of the preceding claims, further comprising:

receiving, from the speaker (10) and/or a receiver, feedback on the speech output (42) through multiple modalities comprising audible signals, visible signals, and/or tactile signals.

8. Computer program product comprising computer readable instructions, which when loaded and run in a computer system, causes the computer system to perform operations according to a method of any one of the preceding claims.

9. Computer system for automatic transformation of myoelectric signals into speech output (42) corresponding to audible speech (50), the system comprising:

a capturing device (26) operable to capture, from a human speaker (10), at least one myoelectric signal (21) representing speech;
a silent/audio conversion component (30) operable to convert at least part of the myoelectric signal (21) to one or more speech features (33); and
a vocoding component (40) operable to vocode the speech features (33) to generate and output the speech output (42) corresponding to the myoelectric signal (21).

10. The system according to claim 9, wherein a representation of the one or more speech features is a spectral representation of speech comprising spectral features, temporal features, and/or spectro-temporal features.

11. The system according to claim 10, wherein the silent/audio conversion component (30) is further operable to compute an estimate for a fundamental frequency of the spectral representation of speech.

12. The system according to any one of claims 9 to 11 wherein joint feature vectors resulting from a prior training phase on myoelectric signals (311) and corresponding audible signals (312) are used to predict the representation of speech for the myoelectric signal.

13. The system according to any one of claims 9 to 12, wherein the at least one myoelectric signal (21) is captured from the speaker's (10) head, throat, face, mouth, chest, and/or neck using an array-based electrode system (26, 261, 262).

14. The system according to any one of claims 9 to 13, wherein the speech is produced by the speaker (10) comprising normally articulated speech, whispered speech, murmured speech, speech that is barely or not audible to a bystander, and/or silently mouthed speech.

15. The system according to any one of claims 9 to 14, wherein the vocoding component (40) is further operable to

receive, from the speaker (10) and/or a receiver, feedback on the speech output (42) through multiple modalities comprising audible signals, visible signals, and/or tactile signals.

Figure 1

# Figure 2

22

10

261 — Biploar electrodes Or Arrays Or Implants

262 — Unipolar electrodes or Arrays Or Implants

263 — Reference electrodes

26

21

221 — Initial Noise Filtering (10 Hz Highpass)

222 — Differential Amplification

223 — Second stage Filtering

224 — Transmission Microcontroller

23 — Digital Signal 16bit resolution 1000 Hz Sampling rate

# Figure 3

**Figure 4A**

**Figure 4B**

Figure 4C

**Figure 5**

Figure 6

# Figure 7

34

311

EMG signals

Trained GMMs for
Spectral Speech
Representation

313

315

EMG recording

Acoustic
speech

Speech recording

Speech
signals

SVM
classifier for
Voicing

Trained GMMs for
Fundamental
Frequency

10

312

314

**Figure 8**

Training Part

Reference    U    V    U    V

310    Training → SVM  314

Voiced Frames    Training →    GMM  34

Conversion Part  311

EMG Data → SVM  314 → Estimated U/V information

GMM  34 → Estimated F0 Contour

**Figure 9**

EP 2 887 351 A1

**EP 2 887 351 A1**

Europäisches Patentamt
European Patent Office
Office européen des brevets

## EUROPEAN SEARCH REPORT

Application Number

EP 13 00 5905

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Arthur R Toth ET AL: "Synthesizing Speech from Electromyography using Voice Transformation Techniques", Interspeech 2009, 6 September 2009 (2009-09-06), pages 652-655, XP055110725, Brighton Retrieved from the Internet: URL:https://www.cs.cmu.edu/afs/cs.cmu.edu/Web/People/atoth/papers/IS090521.pdf [retrieved on 2014-03-28] | 1-6,8-14 | INV. G10L25/75 G10L13/02 |
| Y | * abstract * * Sections 2, 3, 4 and 5; table 1 * | 7,15 | |
| X | DENBY B ET AL: "Silent speech interfaces", SPEECH COMMUNICATION, ELSEVIER SCIENCE PUBLISHERS, AMSTERDAM, NL, vol. 52, no. 4, 1 April 2010 (2010-04-01), pages 270-287, XP026917071, ISSN: 0167-6393, DOI: 10.1016/J.SPECOM.2009.08.002 [retrieved on 2009-08-27] | 1,8,9 | |
| A | * Section 3 * | 2-6, 10-14 | |

TECHNICAL FIELDS SEARCHED (IPC)

G10L

-/--

The present search report has been drawn up for all claims

| Place of search | Date of completion of the search | Examiner |
|---|---|---|
| Munich | 4 November 2014 | Müller, Achim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

29

Europäisches
Patentamt

European
Patent Office

Office européen
des brevets

## EUROPEAN SEARCH REPORT

Application Number

EP 13 00 5905

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TODA ET AL: "Statistical mapping between articulatory movements and acoustic spectrum using a Gaussian mixture model", SPEECH COMMUNICATION, ELSEVIER SCIENCE PUBLISHERS, AMSTERDAM, NL, vol. 50, no. 3, 29 January 2008 (2008-01-29), pages 215-227, XP022436797, ISSN: 0167-6393, DOI: 10.1016/J.SPECOM.2007.09.001 | 1,8,9 | |
| A | * Sections 1 to 3 * | 2-6, 10-14 | |
| | ----- | | |
| X | EP 1 374 765 A1 (NTT DOCOMO INC [JP]) 2 January 2004 (2004-01-02) | 1,8,9 | |
| A | * figure 5 * | 2-6, 10-14 | |
| | ----- | | |
| A | NOBORU SUGIE ET AL: "A Speech Prosthesis Employing a Speech Synthesizer-Vowel Discrimination from Perioral Muscle Activities and Vowel Production", IEEE TRANSACTIONS ON BIOMEDICAL ENGINEERING, IEEE SERVICE CENTER, PISCATAWAY, NJ, USA, vol. BME-32, no. 7, 1 July 1985 (1985-07-01), pages 485-490, XP011265674, ISSN: 0018-9294, DOI: 10.1109/TBME.1985.325564 * the whole document * | 1-6,8-14 | TECHNICAL FIELDS SEARCHED (IPC) |
| | ----- | | |
| Y | US 2010/312564 A1 (PLUMPE MICHAEL D [US]) 9 December 2010 (2010-12-09) * paragraph [0002] - paragraph [0003] * * paragraph [0026] - paragraph [0030] * | 7,15 | |
| | ----- | | |
| | -/-- | | |

The present search report has been drawn up for all claims

| Place of search | Date of completion of the search | Examiner |
|---|---|---|
| Munich | 4 November 2014 | Müller, Achim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 13 00 5905

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 6 081 780 A (LUMELSKY LEON [US]) 27 June 2000 (2000-06-27) * column 12, line 31 - column 14, line 14 * | 7,15 | |
| A | US 2010/312565 A1 (WANG JIAN-CHAO [CN] ET AL) 9 December 2010 (2010-12-09) * figure 2 * | 7,15 | |

TECHNICAL FIELDS
SEARCHED      (IPC)

The present search report has been drawn up for all claims

| Place of search | Date of completion of the search | Examiner |
|---|---|---|
| Munich | 4 November 2014 | Müller, Achim |

EPO FORM 1503 03.82 (P04C01)

Europäisches
Patentamt

European
Patent Office

Office européen
des brevets

Application Number

EP 13 00 5905

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

Application Number

EP 13 00 5905

The Search Division considers that the present European patent application does not comply with the requirements of unity of invention and relates to several inventions or groups of inventions, namely:

1. claims: 1-6, 8-14

   Method for automatic transformation of myoelectric signals into speech output.
   ---

2. claims: 7, 15

   Receiving feedback on speech output through multiple modalities comprising audible signals, visible signals, and/or tactile signals.
   ---

**ANNEX TO THE EUROPEAN SEARCH REPORT
ON EUROPEAN PATENT APPLICATION NO.**

EP 13 00 5905

This annex lists the patent family members relating to the patent documents cited in  the above-mentioned European search report.
The members are as contained in the European Patent Office EDP file on
The European Patent Office is in no way liable for these particulars which are merely given for the purpose of information.

04-11-2014

| Patent document cited in search report | | Publication date | Patent family member(s) | | Publication date |
|---|---|---|---|---|---|
| EP 1374765 | A1 | 02-01-2004 | CN | 1471334 A | 28-01-2004 |
| | | | DE | 60316072 T2 | 29-05-2008 |
| | | | EP | 1374765 A1 | 02-01-2004 |
| | | | JP | 2004016658 A | 22-01-2004 |
| | | | US | 2004034645 A1 | 19-02-2004 |
| US 2010312564 | A1 | 09-12-2010 | NONE | | |
| US 6081780 | A | 27-06-2000 | NONE | | |
| US 2010312565 | A1 | 09-12-2010 | NONE | | |

EPO FORM P0459

For more details about this annex : see Official Journal of the European Patent Office, No. 12/82